# EUROPEAN PATENT SPECIFICATION

(11) **EP 2 796 113 B1**
(45) Date of publication and mention of the grant of the patent: **03.08.2016**
(21) Application number: 14165773.4
(22) Date of filing: 24.04.2014
(51) Int. Cl.: A61F 5/01, A61F 5/02, A61F 5/05, A61F 5/14, A61F 5/32

(54) **Device for supporting at least a part of the body of a person and method for adjusting such a device**
Vorrichtung zur Unterstützung mindestens eines Körperteils einer Person und Verfahren zur Anpassung einer solchen Vorrichtung
Dispositif pour supporter au moins une partie du corps d'une personne et procédé de réglage d'un tel dispositif

(30) Priority: 24.04.2013 NL 2010699
(43) Date of publication of application: 29.10.2014
(73) Proprietor: P.R. Sella B.V., 7575 BS Oldenzaal (NL)
(72) Inventor: Huttenhuis, Alouisius Gerardus, 7591 PZ Denekamp (NL); Huttenhuis, Tijn Pieter Lodewijk, 7571 BA Oldenzaal (NL)
(74) Representative: Slikker, Wilhelmina Johanna

(56) References cited:
- DE-A1- 2 250 681
- US-A- 4 828 325
- US-A- 4 903 690
- US-A- 5 449 478
- US-A1- 2012 296 249
- US-B1- 6 490 730

## Description

The invention relates to a device for supporting at least a part of the body of a person. The invention relates particularly to an orthosis.

Orthoses for supporting a part of the body of a person are per se known. Such an orthosis can be a pre-formed device which can be used for different persons, or an individual made-to-measure orthosis. A drawback of the pre-formed orthosis is that the part of the body of the person is supported less well than is the case with an orthosis made to size. A drawback in the production of the made-to-measure orthosis is that a great deal of workmanship, expertise and skill is required for the purpose.

It is an object of the invention to obviate at least one of the above stated drawbacks of the per se known orthosis. It is a particular object of the invention to provide an orthosis for supporting at least a part of the body of a person which provides good support for the part of the body and/or can be realized relatively efficiently.

An orthosis of the type stated in the preamble of claim 1 is disclosed in US 5 449 478.

The above object is solved by an orthosis as defined in claim 1.

An advantage of the orthosis according to the invention is that the casing is a standard product suitable for different persons, while the deformable element disposed in the casing can be adjusted to an individual by shaping the deformable element to the part of the body of the person in question. The orthosis can hereby be prefabricated and adapted to the relevant person at another location. The orthosis hereby provides the advantage that it can be produced efficiently and provide customized selective support to the part of the body of the person. The invention particularly makes it possible to have a part of the supporting element fit to the individual curvature of parts of the body. The part of the body of the person preferably supports against the device during deforming of the deformable element so that the deformable element can shape itself almost continuously to the part of the body. Following curing of the curable medium the deformable element is fixed in its deformed shape and can then provide good support to the part of the body. The deformable element is therefore adjustable between a first deformable state and a second deformed, fixed state. The deformable element can either be already filled with the curable medium, which has to be activated for curing and therefore for adjustment to the second state, or be hollow and filled for adjustment purposes with a curable medium which cures almost immediately after being introduced into the deformable element. The medium optionally expands after being introduced or activated and before curing.

The orthosis according to the invention can particularly be a seat of a seating device, such as in particular a (wheel)chair. It has been found by applicant that such a seat orthosis according to the invention provides good, selective support to a seated person.

The invention therefore relates particularly to a seat orthosis for supporting the buttocks and at least a part of the upper legs of a person.

It is noted that the deformable element can only be disposed in a part of the volume of the casing, wherein no deformable element is present in the other part of the volume. The orthosis here provides support and/or correction for the part of the body which is in register with the one part of the volume, and thereby with the cured deformable element, while conversely another part of the body which is in register with the other part of the volume is relieved of load. Such a deformable element which only takes up a part of the volume of the casing and is not disposed in the other part of the volume is hereby a preferred embodiment because selectively determined parts of the body can hereby be loaded or, conversely, relieved of load. This is not possible with deformable elements which are disposed in substantially the whole volume of the casing since they do not allow selective loading or relieving of load because they support or correct substantially the whole part of the body.

In the other part of the volume two opposite sides of the casing lie directly against each other, at least during use, without the deformable element extending therebetween. The one part of the volume and the other part of the volume are therefore particularly understood to mean a part and another part of the contact surface of the casing, in which contact surface the part of the body lies against the casing during use. According to the invention, the deformable element is thus disposed over only a part of the contact surface of the casing, wherein no deformable element is present over the other part of the contact surface. Lying against the one part of the contact surface where the deformable element is disposed is a first part of the part of the body, this one part of the contact surface being in contact or in register with the deformable element. This first part of the part of the body is therefore supported or corrected by the deformable element, or at least loaded thereby. Lying against the other part of the contact surface where no deformable element is disposed is a second part of the part of the body, this other part of the contact surface being in direct contact with the opposite side of the casing without the deformable element extending therebetween. This second part of the part of the body is therefore not supported or corrected by the deformable element, and is particularly relieved of load.

The ratio of the one part of the contact surface and the other part of the contact surface can be chosen as desired. The deformable element can for instance take up 10%, 20%, 30%, 40%, 50%, 60%, 70%, 80% or 90% of the total surface area, whereby the other part of the volume takes up respectively 90%, 80%, 70%, 60%, 50%, 40%, 30%, 20% or 10% of the total surface area. It is particularly noted that the other part of the contact surface is not a part left clear unintentionally but a part left clear intentionally, of a reasonable size of for instance said minimum of 10%, 20%, 30%, 40%, 50%, 60%, 70%, 80% or 90% of the total contact surface area, in order to thus relieve load selectively.

Areas which may be suitable for intentional relieving of load are at the position of the coccyx, tuberosities, trochanters, pubic bone and pubic area.

It is also noted that the person can assume any suitable position while being supported, such as seated, standing or recumbent, depending on the type of orthosis. The orthosis can support the person temporarily, for instance during the process of recovery from a fracture or bruising, or substantially permanently.

As described above, the deformable element can be a deformable element which is fillable with a curable medium. It is practical for this purpose for the deformable element to have a filling opening for filling of the deformable element with the medium. The filling opening can optionally comprise a hose coupled thereto for the purpose of connecting the filling opening to a filling gun or the like filled with the medium. The filling opening can alternatively comprise connecting means or be embodied such that a hose of a filling gun or the like is connectable thereto. Upstream and/or downstream of the filling opening as seen in the feed direction of the medium can be provided a non-return valve which can be disposed in an optional hose and/or the deformable element. Such a non-return valve allows the medium to be fed to the deformable element but prevents the medium flowing back.

In an embodiment of the orthosis according to the invention the medium curable by activation consists of at least two components, which two components are arranged separately of each other in the deformable element and wherein activation takes place by bringing the two components into contact with each other. The two components can react with each other when they come into mutual contact, after which they expand and cure. Separation of the two components can for instance take place by arranging the two components in two different areas of the deformable element, wherein the two areas are separated from each other. The orthosis can for instance comprise a clamp which is disposed in a central part between the two areas and there clamps the deformable element together so that the components cannot move past the clamp to the other area. Activation can then take place by removing the clamp and optionally actively mixing the two components.

In another embodiment of the orthosis according to the invention the deformable element comprises a seed crystal which is arranged in the deformable element or can be produced therein. The seed crystal can be activated or produced, for instance by buckling a metal plate or pressing a button, wherein the medium will crystallize and cure.

The medium is for instance chosen from the group comprising polyurethane (PUR) and a sodium acetate solution in water. When introduced into the deformable element or when activated by for instance bringing the two components polyol and isocyanate into contact with each other, PUR will expand and cure, wherein the PUR shapes itself substantially continuously to the part of the body of the person. The sodium acetate solution will crystallize following activation by means of the buckling plate, and cure. The curing of each of the two media after introduction or activation is largely realized for instance after several minutes, for instance ten minutes, or at least realized to an extent such that the shape cannot be deformed any further. A catalyst, for instance benzyldimethylamine (BDMA), can optionally be added to the PUR in order to accelerate this process so that the deformable element is shaped more quickly to the part of the body and the person need not sit, lie or stand immobile for a very long time or wait for a very long time. An advantage of the sodium acetate solution in water is that, when heated to above its melting point, it becomes liquid again and can thereby be deformed once again.

In another embodiment of the orthosis according to the invention the casing comprises a pressure-distributing element. Such a pressure-distributing element can be a resiliently compressible element, such as a soft foam element or foam cushion. The thickness of the pressure-distributing element can be different at different locations, depending on the desired pressure distribution on the part of the body. As described above, the pressure-distributing element can be a standard element which can be suitable for different people with more or less the same posture. Only a number of pressure-distributing elements need thus be provided for a number of different postures, this saving costs compared to making such a pressure-distributing element to size for an individual.

It is noted that an outer surface of the pressure-distributing element forms the above described contact surface of the casing.

In practical manner the casing also comprises a stiff element which is connected or connectable to the pressure-distributing element, wherein the deformable element is disposed or can be disposed between the pressure-distributing element and the stiff element. In the case of such a casing the pressure-distributing element is oriented during use toward the person and the stiff element is disposed on a side remote from the person. This stiff element can be pre-formed for the purpose of providing an initial support to the part of the body, wherein the deformable element disposed between the pressure-distributing element and the stiff element shapes itself individually to the part of the body and so provides made-to-measure support. The stiff element can be a standard element suitable for a number of people with more or less the same posture.

When a deformable element is used which is disposed over only a part of the contact surface and not in the other part, the pressure-distributing element lies directly against the stiff element in the other part, without the deformable element extending therebetween. The stiff element can thus provide the initial support to particularly the above described second part of the part of the body, and in particular relieve the load on the second part of the part of the body.

It will therefore be apparent that in said preferred embodiment the orthosis has a structure in the one part of the contact surface consisting, as seen in depth direction, of the pressure-distributing element, the deformable element and the stiff element and that in the other part of the contact surface the orthosis has a structure consisting, as seen in the depth direction, of the pressure-distributing element and the stiff element. It is noted that this does not preclude optional layers other than the stated layers being provided.

In yet another embodiment of the orthosis according to the invention the deformable element has a shape adapted to the part of the body of the person. An expert can selectively determine the dimensions, shape and location of the deformable element in the casing. This is possible by making a selection from a range of deformable elements suitable for providing support to different body parts or providing support in the case of different medical disorders, or possibly by manufacturing the deformable element wholly to specification. The deformable element can here be an element adapted to the person or made to size, while the casing is a standard element. Providing a deformable element with a shape adapted to the person makes it possible to provide still better individual support to the part of the body of the person.

In yet another embodiment of the orthosis according to the invention the deformable element comprises two sides which are disposed opposite each other and connected to each other along their periphery. The connection can for instance be realized by a peripheral seam or by folding a sheet-like material, wherein the folded part mutually connects the two sides.

The two sides can be connected to each other at determined locations along the surface of the two sides. An advantage hereof is that the thickness of the deformable element after expansion and/or the shape of the deformable element after expansion can hereby be controlled.

In yet another embodiment of the orthosis according to the invention the element has at least one peripheral pleat. An advantage hereof is that the thickness of the deformable element after expansion and/or the shape of the deformable element after expansion can hereby be controlled.

In practical manner an inward directed end of the pleat can be connected to a part of the deformable element. This prevents the pleat being pressed outward when the medium expands. The connection can for instance be effected by a string or strap, wherein the string or strap is connected on one side to the inward directed end of the pleat and on the other to another suitable part of the inner surface of the deformable element.

The deformable element can in practical manner have at least one air outlet opening. Air which may be present in the deformable element can leave the element via the air outlet opening during filling or expanding of the medium so that the element is properly filled with the expanded, cured element and can therefore provide good support. The air outlet opening preferably has dimensions such that the air can exit therethrough, but the medium cannot. The element comprises for instance a number of very small perforations.

In yet another embodiment of the orthosis according to the invention the orthosis comprises attaching means for attaching the deformable element to the casing such that the deformable element can be disposed fixedly at a determined position in the casing. An advantage hereof is that the deformable element can be held at a determined location in the casing so that the deformable element can support the part of the body correctly. This is particularly advantageous in the case of a deformable element which does not take up the full volume of the casing but only a part thereof.

The attaching means can for instance be selected from the group comprising glue and velcro tape. An advantage of velcro tape is that the deformable element is fixedly disposed in releasable manner and can if desired be released and disposed at another location.

Alternatively or additionally, the deformable element can have a discharge opening for discharging a part of the medium such that medium carried via the discharge opening out of the deformable element attaches the deformable element to the casing. The discharge opening preferably has dimensions here such that only a small part of the medium can flow out and subsequently adheres by curing to the casing. The opening can optionally be closed prior to filling of the deformable element with the medium or prior to activation of the deformable element.

The invention also relates to a method for adjusting an orthosis for supporting at least a part of the body of a person, as defined in claim 13.

The orthosis according to the invention can be adjusted, i.e. adapted to a person, in simple manner using the method according to the invention.

Step (b) and/or step (c) is preferably performed while the part of the body of the person supports against the orthosis. The deformable element hereby shapes itself to the part of the body and can provide good support following curing.

As described above, the medium can be selected from the group comprising polyurethane (PUR) and a sodium acetate solution.

The invention will be further elucidated with reference to figures shown in a drawing, in which:
figures 1A-1D show the orthosis according to a first embodiment of the invention, wherein figures 1A and 1B show a perspective exploded view of the orthosis at two different stages, figure 1C shows a longitudinal section of the orthosis and figure 1D shows a cross-section of the orthosis;
figures 2A-2F show different forms of the deformable element according to the invention;
figures 3A and 3B show respectively a cross-section and a perspective view of the deformable element according to a second embodiment of the invention;
figures 4A and 4B show respectively a cross-section and a perspective view of the deformable element according to a third embodiment of the invention;
figures 5A and 5B show respectively a cross-section and a perspective view of the deformable element according to a fourth embodiment of the invention;
figures 6A and 6B show respectively a top view and a cross-section of the deformable element according to a fifth embodiment of the invention;
figures 7A and 7B show respectively a top view and a cross-section of the deformable element according to a sixth embodiment of the invention;
figures 8A-8C show respectively a top view, a cross-section and an exploded view of the orthosis according to a second embodiment of the invention;
figures 9-13 show embodiments of an orthosis that are not part of the invention;
figures 14A-14C show a deformable element according to a third embodiment of the invention, wherein figures 14A and 14B show the deformable element in two states and wherein figure 14C shows an exploded view of the orthosis with this deformable element, and
figure 15 shows an exploded view of the orthosis according to the invention with a deformable element according to a fourth embodiment of the invention.

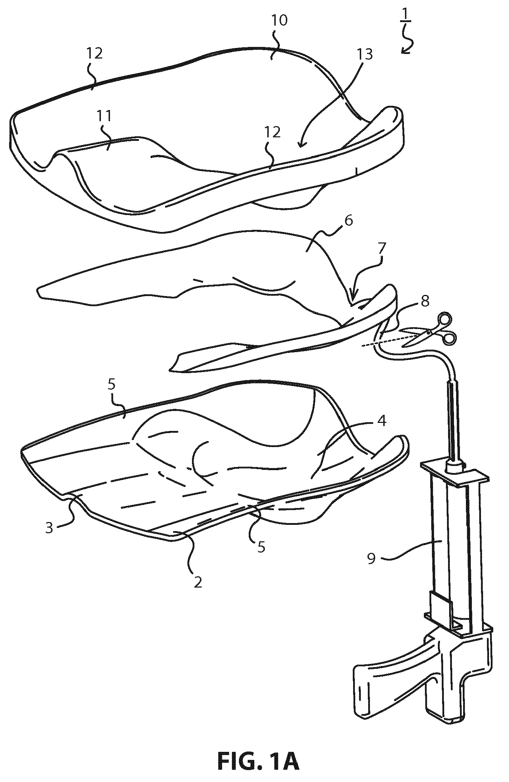

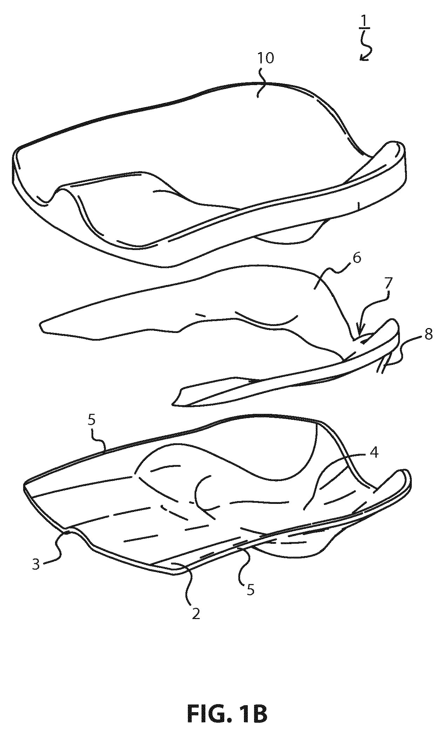

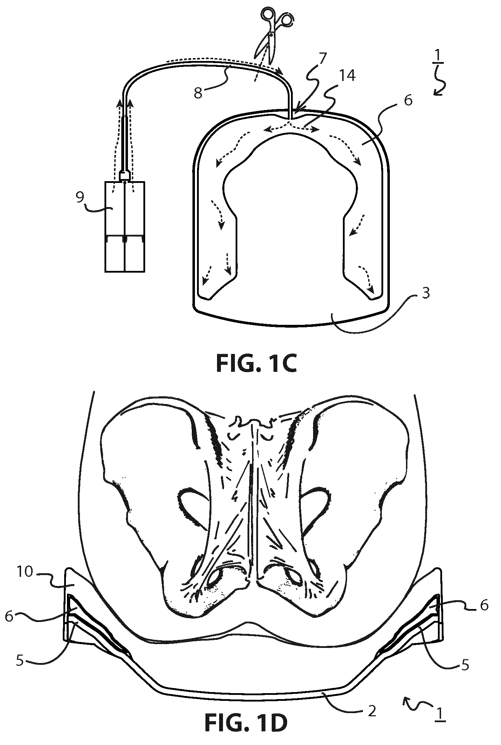

Figures 1A-1D show a seat 1 for supporting the buttocks and at least a part of the upper legs of a seated person. Seat 1 comprises a stiff shell 2 manufactured from for instance a plastic. Shell 2 is designed such that it supports the buttocks and the upper legs of the person. Shell 2 has for this purpose a raised portion 3 extending along the longitudinal central axis over a foremost part of shell 2, whereby it extends during use between the upper legs of the seated person and thus supports the inner sides of the upper legs. The longitudinal side edges 5 of the hard shell 2, which are located during use on the outer sides of the upper legs of the seated person, are raised so that shell 2 supports the outer sides of the upper legs. Arranged in a rear part of shell 2 is a centrally disposed deepened portion or recess 4 which is located during use under the tuberosities and the coccyx of the seated person in order to relieve the load on tuberosities to limited extent and to wholly relieve the load on the coccyx.

A deformable element 6 according to the invention is arranged on shell 2. Deformable element 6 has a filling opening 7 to which a hose 8 is connected. Hose 8 can be coupled to a filling gun 9 for the purpose of filling the cavity of deformable element 6 with a curable filler. The filler is for instance a curable foam such as polyurethane (PUR). The filler for instance comprises two materials which are arranged separately of each other in filling gun 9 and which come together during filling of deformable element 6 and there react with each other such that they optionally foam and then cure. The flow of the filler in deformable element 6 is indicated with arrows 14. Deformable element 6 has a form such that, following filling and curing, it provides selective support to the buttocks and upper legs of the seated person. Hard shell 2 can in this way have a more or less standard form, wherein the seat can be made to size for an individual by selecting a deformable element 6 of suitable shape. The seat according to the invention can hereby function as an orthosis by selectively supporting and correcting parts of the seated person. Deformable element 6 in figures 1A-1D is disposed solely along the periphery of shell 2, so leaving clear the front side of seat 1. Once this deformable element 6 has been filled, extra support is imparted to the outer sides of the upper legs and the rear side of the buttocks of the seated person. This selective support on the outer sides of the upper legs and the rear side of the buttocks of the seated person can be provided because deformable element 6 is disposed only in this one part of the surface of shell 2, while no deformable element is disposed in the other part of the surface of shell 2, so that the remaining parts of the upper legs and the buttocks are supported directly by shell 2 and not by a deformable element. Deformable element 6 can be manufactured from any flexible material. The deformable element can for instance be made of a plastic foil or a fabric, such as optionally coated textile. The advantage of a fabric is that it is permeable to air but not to the curable medium. The material can also be an incontinence material.

Disposed on top of shell 2 and deformable element 6 is a soft pressure-distributing cushion 10, for instance manufactured from a flexible, to some extent compressible foam. Cushion 10 provides for a pleasant seating experience. Cushion 10 has a form adapted to shell 2 with a raised central edge 11 in a front part of cushion 10, standing side edges 12 and a central recess 13 arranged in the rear part.

Filling of deformable element 6 preferably takes place while the person is sitting on seat 1 such that the foam shapes itself to the contours of the seated person and can thus provide optimal support after curing. Hose 8 can be cut following curing. The cutting preferably takes place as close as possible to filling opening 7 so that the hose extends over only a short length once it has been severed.

It is noted that a number of different shells 2 and cushions 10 can for instance be manufactured with different dimensions, wherein each dimension is suitable for a person with measurements lying within a determined range. Only a number of different shells 2 and cushions 10 in this way need be manufactured which, through a correct selection of a suitable deformable element 6 of a suitable shape, can provide the correct support to any individual.

Is also noted that filling opening 7 need not be fixedly connected to a hose. It is likewise possible for a releasable hose to be connected to the filling opening. The filling opening can for this purpose optionally have connecting means for connection of the hose.

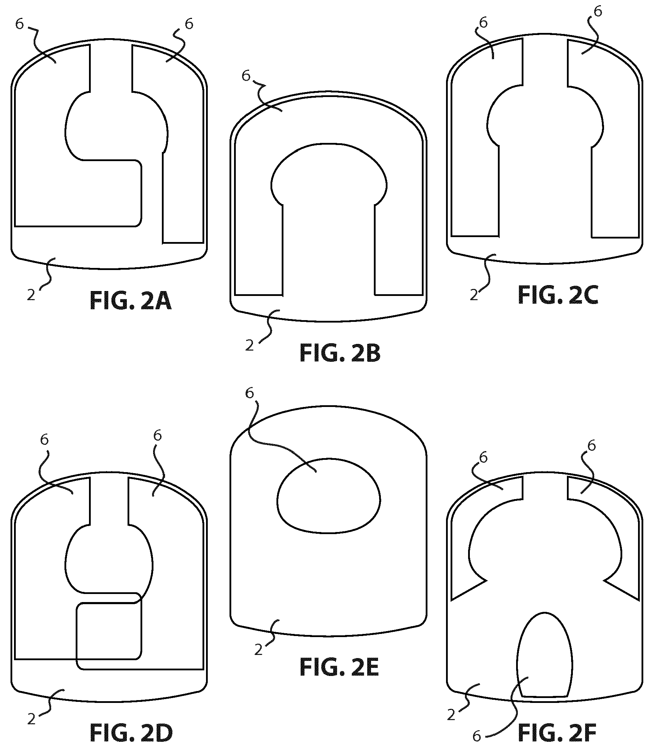

Figures 2A-2F show different forms of deformable element 6. In figure 2A two deformable elements 6 are disposed on shell 2. Disposed on the one side of shell 2 is an element 6 which extends from the rear side of shell 2 along the periphery of shell 2 to the front side of shell 2 and which extends inward in the area of the front side of shell 2. This element 6 provides support on the outer side of the right buttock and is particularly suitable for a person with an amputated right leg. Element 6 disposed on the other side of shell 2 extends from the rear side of shell 2 along the periphery of shell 2 to the front side of shell 2. This element 6 provides support to the outer side of the left buttock and left upper leg of the seated person.

Figure 2B shows a single deformable element 6 extending along the periphery of shell 2 while leaving clear the front side of shell 2. This element 6 corresponds in the main to element 6 of figures 1A-1D. This deformable element 6 provides support to the outer sides of the buttocks and upper legs of the seated person.

Figure 2C shows two elements 6, each extending from the rear side of shell 2 along the periphery of shell 2 to the front side of shell 2. Elements 6 provide support to the outer sides of the buttocks and upper legs of the seated person, wherein the rear side of the buttocks is relieved of load.

Figure 2D shows two deformable elements 6 which each extend from the rear side of shell 2 along the periphery of shell 2 to the front side of shell 2, and which extend inward in the area of the front side of shell 2. The inward extending parts of elements 6 partially overlap each other.

Figure 2E shows a single deformable element 6 disposed over the surface of shell 2 in a central part in the rear part of shell 2. During use the position of deformable element 6 corresponds to the position of the tuberosities, i.e. this deformable element 6 is in register with recess 4 of shell 2.

Figure 2F shows three elements 6, wherein two elements 6 are disposed in the rear part of shell 2 on either side of shell 2 and along the periphery of shell 2. These elements 6 support the outer sides of the buttocks of the seated person. In the front part of shell 2 the third element 6 is disposed along the longitudinal central axis of shell 2. This element 6 supports the inner sides of the upper legs of the seated person.

Figures 2A-2F show that any desired number of deformable elements 6 of any desired shape can be provided in order to impart suitable support to the seated person. All deformable elements 6 of figures 2A-2F have in common that they are disposed over only a part of the surface of cushion 10 and shell 2, and leave another part clear. In this one part the seat has a structure, as seen in depth direction, of cushion 10, pressure-distributing element 6 and shell 2. In the other part cushion 10 and shell 2 directly adjoin each other without a pressure-distributing element extending therebetween, whereby the structure of the other part, as seen in the depth direction, is cushion 10 and shell 2.

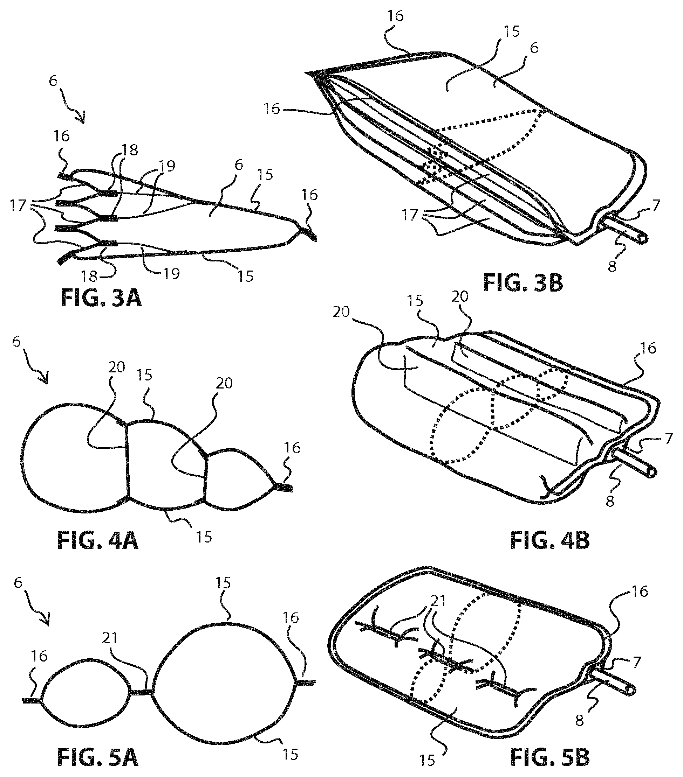

Figures 3A and 3B show a deformable element 6 which comprises two sides 15 disposed opposite each other and which are connected to each other along their periphery by means of peripheral seams 16. On one side of element 6 the periphery of element 6 comprises three folds or pleats 17. The inward directed ends 18 of pleats 17 are connected to the underside or upper side of deformable element 6 using a string or strap 19. During filling of deformable element 6 as according to figures 3A and 3B with the expanding medium the pleats 17 will displace away from each other. An element 6 is in this way obtained with relatively thick filling in the area of pleats 17. Because ends 18 are connected to the underside or upper side of element 6, they will remain in place during expansion of the medium and will not displace outward, or only do so to limited extent. Element 6 has a filling opening 7 in which an end zone of a hose 8 is arranged.

Figures 4A and 4B show a deformable element 6 comprising two sides 15 which are disposed opposite each other and which are connected to each other along their periphery by means of peripheral seams 16. No peripheral seam is present on one side of element 6. On this one side the two sides 15 are mutually connected in that element 6 consists of one sheet-like element which is folded over in the area of the one side in order to form the two sides 15. The two sides 15 are connected to each other at two locations along the surface of the two sides 15 by walls 20 extending substantially perpendicularly between the two sides 15 over a length of element 6. Walls 20 limit the maximum distance between the two sides 15 during expansion of the medium, whereby the thickness of element 6 is limited. In element 6 as according to figures 4A and 4B the two walls 20 each have a different height, whereby after filling and curing the element 6 has three zones, each of a different thickness. The thickness of the zones can be chosen by selecting a wall of a suitable height. Close to the periphery of element 6 the walls 20 are not connected to the two sides 15 such that between walls 20 and the periphery of element 6 a space is left clear through which the medium can pass in order to fill the three zones separated by the two walls 20. Element 6 has a filling opening 7 in which an end zone of a hose 8 is arranged.

Figures 5A and 5B show a deformable element 6 comprising two sides 15 which are disposed opposite each other and which are connected to each other along their periphery by means of peripheral seams 16. The two sides 15 are directly connected to each other by seams 21 at three locations along the surface of the two sides 15. Seams 21 limit the maximum distance between the two sides 15 during expansion of the medium, whereby the thickness of element 6 is limited. The distance between the periphery of element 6 and seams 21 differs on either side of seams 21, whereby the two zones bounded by peripheral seams 16 and seams 21 each have a different thickness. The thickness of element 6 can be chosen by selecting a suitable distance between two adjacent seams. Element 6 has a filling opening 7 in which an end zone of a hose 8 is arranged.

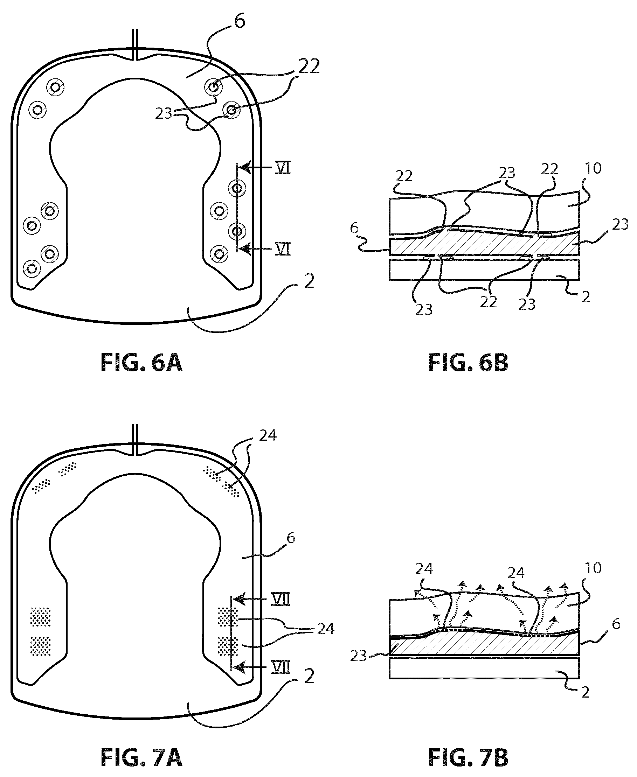

Figures 6A and 6B show a deformable element 6 with a number of discharge openings 22. Discharge openings 22 have dimensions such that during filling of element 6 with medium 23 a part of the medium 23 is discharged from the cavity of element 6 via openings 22. The medium 23 discharged from openings 22 will adhere during curing to shell 2 and cushion 10 on the sides of element 6 facing respectively toward shell 2 and cushion 10. A connection between element 6 and shell 2 and cushion 10 is in this way provided in simple manner during filling of element 6, so that element 6 is no longer displaceable after curing.

Figures 7A and 7B show a deformable element 6 with a number of perforations 24. Perforations 24 have dimensions such that medium 23 cannot flow therethrough. Air which may be present in element 6 can however escape from element 6 via perforations 24. This enables better filling of element 6 and/or prevents element 6 tearing during expansion of medium 23.

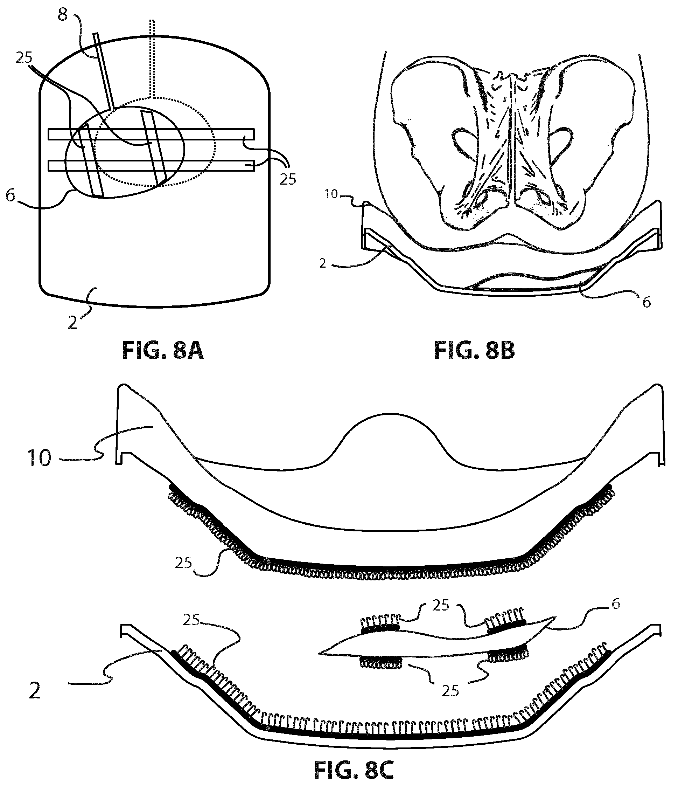

Figures 8A-8C show a seat 1, wherein strips of velcro tape 25 are arranged on the inner surfaces of shell 2 and cushion 10 and on the outer surfaces of deformable element 6. The strips of velcro tape 25 on shell 2 and cushion 10 have loops, while the strips of velcro tape 25 on element 6 have hooks, which hooks can engage in the loops in order to realize a connection between element 6 and shell 2 and cushion 10. It will be apparent that it is also possible for element 6 to have the loops and shell 2 and cushion 10 to have the hooks. Using the strips of velcro tape 25 on shell 2 and cushion 10, element 6 can be arranged and held in place at any desired position in the area of the strips of velcro tape 25. Because the attachment takes place with velcro tape, the attachment is releasable, whereby it is possible to release element 6 from shell 2 and cushion 10 and optionally arrange it at another location. Figure 8A shows two possible positions of element 6 relative to shell 2 and cushion 10.

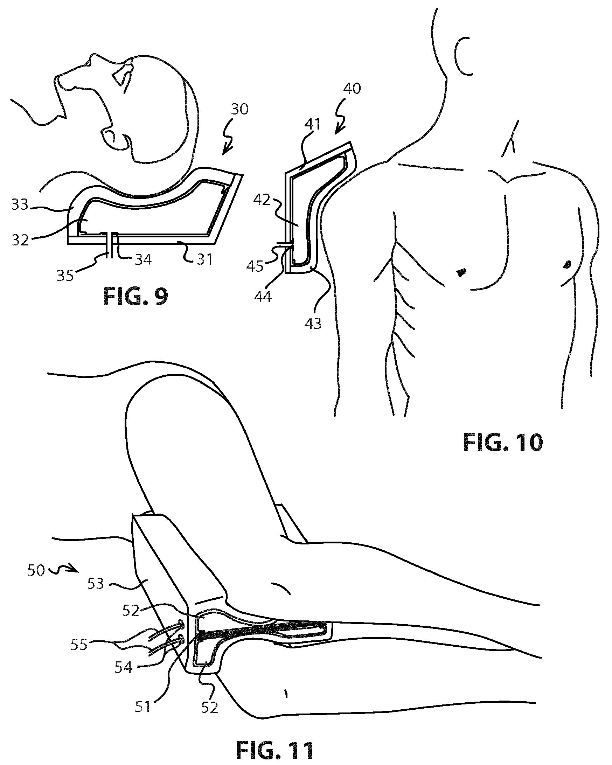

Figures 9-13 show different types of orthosis for supporting various parts of the body of a seated or recumbent person. Figure 9 thus shows a head orthosis 30. Head orthosis 30 comprises a stiff shell 31, a deformable element 32 disposed on shell 31 and a pressure-distributing cushion 33 arranged on deformable element 32, wherein the head of the recumbent person supports on cushion 33. Deformable element 32 has a filling opening 34 with a hose 35 connected thereto. Similarly to deformable element 6, deformable element 32 can be filled with a curable medium via hose 35 and filling opening 34, preferably while the recumbent person supports his/her head on orthosis 30, so that element 32, and thereby orthosis 30, shapes itself to the contours of the head of the person and can thus provide good support.

Figure 10 shows a shoulder orthosis 40. Shoulder orthosis 40 comprises a stiff shell 41, a deformable element 42 disposed on shell 41 and a pressure-distributing cushion 43 which is arranged on deformable element 42 and against which the shoulder of the seated person supports. Deformable element 42 has a filling opening 44 with a hose 45 connected thereto. Similarly to deformable element 6, deformable element 42 can be filled with a curable medium via hose 45 and filling opening 44, preferably while the seated person supports his/her shoulder against orthosis 40, so that element 42, and thereby orthosis 40, shapes itself to the contours of the shoulder of the person and can thus provide good support.

Figure 11 shows a central abduction orthosis 50. Abduction orthosis 50 comprises a stiff plate-like element 51, deformable elements 52 disposed on either side of element 51 and a pressure-distributing cushion 53 which extends round deformable elements 52 and element 51 and against which the inner sides of the upper legs of the recumbent person support. Both deformable elements 52 have a filling opening 54 with a hose 55 connected thereto. Similarly to deformable element 6, deformable elements 52 can be filled with a curable medium via hose 55 and filling opening 54, preferably while the recumbent person supports his/her upper legs against orthosis 50, so that elements 52, and thereby orthosis 50, shapes itself to the contours of the upper legs of the person and can thus provide good support.

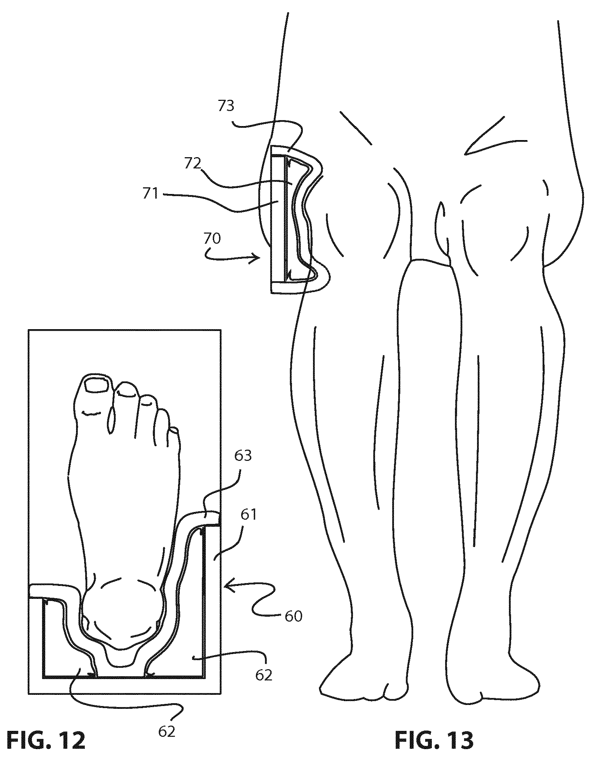

Figure 12 shows a foot orthosis 60. Foot orthosis 60 comprises a stiff shell 61, a deformable element 62 disposed on shell 61 and a pressure-distributing cushion 63 which is arranged on deformable element 62 and against which the foot of the seated or recumbent person supports. Deformable element 62 has a filling opening for filling deformable element 62 with a curable medium via the filling opening (not shown), preferably while the person supports his/her foot against orthosis 60, so that element 62, and thereby orthosis 60, shapes itself to the contours of the foot of the person and can thus provide good support.

Figure 13 shows a knee orthosis 70. Knee orthosis 70 comprises a stiff shell 71, a deformable element 72 disposed on shell 71 and a pressure-distributing cushion 73 which is arranged on deformable element 72 and against which the knee of the seated person supports. Deformable element 72 has a filling opening for filling deformable element 72 with a curable medium via the filling opening (not shown), preferably while the person supports his/her knee against orthosis 70, so that element 72, and thereby orthosis 70, shapes itself to the contours of the knee of the person and can thus provide good support.

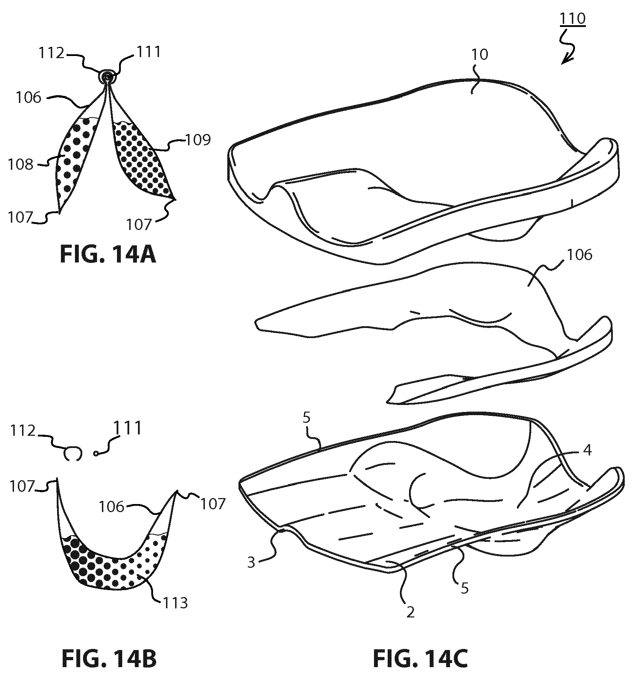

Figures 14A-14C show a seat 110, wherein cushion 10 and shell 2 are the same as those shown in figures 1A-1D. Deformable element 106 differs from the deformable element 6 shown in figures 1A-1D in that deformable element 106 is already filled with PUR, wherein the two components 108, 109 of the PUR, i.e. polyol and isocyanate, are arranged separated from each other in deformable element 106, see figure 14A. The one component 108 is situated here in one part of the volume of deformable element 106, and the other component 109 is situated in another part of the volume of deformable element 106 separated from the first part. The separating system shown in figure 14A comprises a rod 111 and a clamp 112, wherein rod 111 is disposed on the one side of deformable element 106 and clamp 112 is disposed on the other side and clamps around rod 111 such that the two parts of volume 106 are mutually separated. Deformable element 106 is completely closed along its periphery 107 so that the PUR cannot exit therefrom. In figure 14B rod 111 and C-shaped clamp 112 have been removed from deformable element 106 so that the two components 108, 109 can be brought into contact with each other so as to allow the components 108, 109 to react with each other. Mixing of components 108, 109 can be stimulated by kneading the deformable element 106. Once the two components 108, 109 have been brought into contact and optionally actively mixed, deformable element 106 is disposed between shell 2 and cushion 10, see figure 14C. The person will then sit down on cushion 10 in order to individually shape orthosis 110.

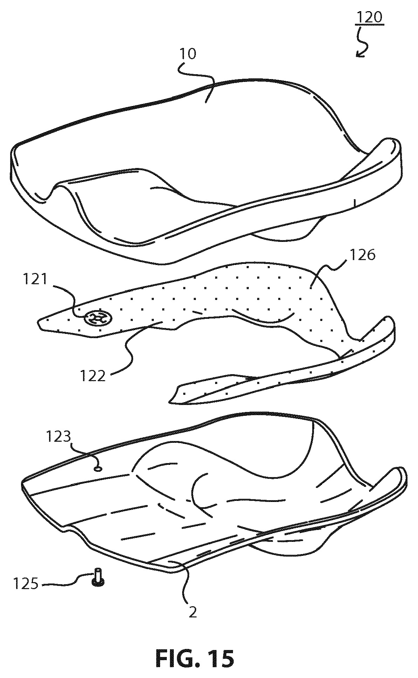

Figure 15 shows an exploded view of a seat 120, wherein cushion 10 and shell 2 are the same as those shown in figures 1A-1D. Deformable element 126 differs from the deformable element 6 shown in figures 1A-1D in that deformable element 126 is already filled with a liquid medium 122, for instance supercooled sodium acetate (CH₃COONa) in water. Also placed in this deformable element 126 is a buckling plate 121 which serves to start a growth core consisting of a number of sodium acetate trihydrate crystals (CH₃COONa.3H₂O), also referred to as nucleus, for the purpose of crystallizing the medium 122. Buckling plate 121 is activated by a button 125 mounted through a hole 123 in seat shell 2. During the curing period, which takes place from formation of the first crystals to the complete crystallization of medium 122, the person is seated on seat 120 in order to individually shape orthosis 120.

With this embodiment it is also possible by means of a reverse reaction to make the crystallized solution liquid again for the purpose of reuse. This is done by heating the crystallized solution above its melting point, which lies around 80°C, for about 10 minutes. An advantage hereof is that it is possible to readjust the deformable element to a part of the body of the person or another person.

It is noted that the invention is not limited to the above discussed exemplary embodiments, but also extends to other variants within the scope of the appended claims.

It will thus be apparent that the orthosis according to the invention is suitable for supporting various parts of the body of a person and is not limited to the shown orthoses.

It will be apparent that it is possible with the orthosis according to the invention to reuse a part or even all of a seat orthosis. This provides the advantage that an already existing orthosis can be adapted with relatively little effort to the changing contour of the body. This can be a result of changes in the clinical picture, body size or desired posture. An individually made provision according to the invention can also be reused for a different person, for instance after a rapid recovery or as a result of death.

## Claims

1. Orthosis for supporting at least a part of the body of a person, comprising:
- a casing (2);
- at least one deformable element (6) disposed in the casing, the deformable element being filled with a medium curable by activation or fillable with a curable medium, for supporting the part of the body of the person after curing of the medium,
**characterized in that**
the casing (2) has a contact surface for lying against the part of the body, wherein the deformable element (6) is disposed over only a part of the contact surface and not over another part thereof.

2. Orthosis as claimed in any of the foregoing claims, wherein the casing comprises a pressure-distributing element.

3. Orthosis as claimed in claim 2, wherein the casing comprises a stiff element which is connected or connectable to the pressure-distributing element, and wherein the deformable element is disposed or can be disposed between the pressure-distributing element and the stiff element.

4. Orthosis as claimed in any of the foregoing claims, wherein the deformable element has a shape adapted to the part of the body of the person.

5. Orthosis as claimed in any of the foregoing claims, wherein the deformable element comprises two sides which are disposed opposite each other and connected to each other along their periphery.

6. Orthosis as claimed in claim 5, wherein the two sides are connected to each other at determined locations along the surface of the two sides.

7. Orthosis as claimed in any of the foregoing claims, wherein the element has at least one peripheral pleat.

8. Orthosis as claimed in claim 7, wherein an inward directed end of the pleat is connected to a part of the deformable element.

9. Orthosis as claimed in any of the foregoing claims, wherein the deformable element has at least one air outlet opening.

10. Orthosis as claimed in any of the foregoing claims, comprising attaching means for attaching the deformable element to the casing such that the deformable element can be disposed fixedly at a determined position in the casing.

11. Orthosis as claimed in claim 10, wherein the attaching means are selected from the group comprising glue and velcro tape.

12. Orthosis as claimed in claim 11, wherein the deformable element has a discharge opening for discharging a part of the medium such that medium carried via the discharge opening out of the deformable element adheres the deformable element to the casing.

13. Method for adjusting an orthosis for supporting at least a part of the body of a person, comprising the steps, to be performed in suitable sequence, of:
(a) providing an orthosis according to any of the claims 1-12, the orthosis comprising:
- a casing;
- at least one deformable element disposed in the casing, the deformable element being filled with a medium curable by activation or fillable with a curable medium, for supporting the part of the body of the person after curing of the medium,
wherein the casing has a contact surface for lying against the part of the body, wherein the deformable element is disposed over only a part of the contact surface and not over another part thereof,
(b) activating the curable medium or filling the deformable element with the curable medium, and
(c) allowing the curable medium to cure.

14. Method as claimed in claim 13, wherein step (b) and/or step (c) is performed while the part of the body of the person supports against the orthosis.

## Patentansprüche

1. Orthese zum Unterstützen mindestens eines Körperteils einer Person, die Folgendes umfasst:
- ein Gehäuse (2);
- mindestens ein verformbares Element (6), das in dem Gehäuse angeordnet ist, wobei das verformbare Element mit einem Medium gefüllt ist, das durch Aktivierung härtbar ist, oder mit einem härtbaren Medium befüllbar ist, um den Körperteil der Person nach dem Härten des Mediums zu unterstützen,
**dadurch gekennzeichnet, dass**
das Gehäuse (2) eine Kontaktoberfläche zum Anliegen an dem Körperteil aufweist, wobei das verformbare Element (6) nur über einem Teil der Kontaktoberfläche und nicht über einem anderen Teil davon angeordnet ist.

2. Orthese gemäß einem der vorhergehenden Ansprüche, wobei das Gehäuse ein druckverteilendes Element umfasst.

3. Orthese gemäß Anspruch 2, wobei das Gehäuse ein steifes Element umfasst, das mit dem druckverteilenden Element verbunden oder verbindbar ist, und wobei das verformbare Element zwischen dem druckverteilenden Element und dem steifen Element angeordnet ist oder angeordnet werden kann.

4. Orthese gemäß einem der vorhergehenden Ansprüche, wobei das verformbare Element eine Form aufweist, die an den Körperteil der Person angepasst ist.

5. Orthese gemäß einem der vorhergehenden Ansprüche, wobei das verformbare Element zwei Seiten umfasst, die einander gegenüber angeordnet sind und miteinander entlang ihres Umfangs verbunden sind.

6. Orthese gemäß Anspruch 5, wobei die zwei Seiten miteinander an vorgegebenen Orten entlang der Oberfläche der zwei Seiten verbunden sind.

7. Orthese gemäß einem der vorhergehenden Ansprüche, wobei das Element mindestens eine periphere Falte aufweist.

8. Orthese gemäß Anspruch 7, wobei ein nach innen gerichtetes Ende der Falte mit einem Teil des verformbaren Elements verbunden ist.

9. Orthese gemäß einem der vorhergehenden Ansprüche, wobei das verformbare Element mindestens eine Luftauslassöffnung aufweist.

10. Orthese gemäß einem der vorhergehenden Ansprüche, die Befestigungsmittel zum Befestigen des verformbaren Elements an dem Gehäuse umfasst, sodass das verformbare Element fixiert an einer vorgegebenen Position in dem Gehäuse angeordnet werden kann.

11. Orthese gemäß Anspruch 10, wobei das Befestigungsmittel ausgewählt ist aus der Gruppe umfassend Klebstoff und Klettband.

12. Orthese gemäß Anspruch 11, wobei das verformbare Element eine Auslassöffnung zum Herauslassen eines Teils des Mediums aufweist, so das Medium, das über die Auslassöffnung aus dem verformbaren Element herausgetragen wird, das verformbare Element mit dem Gehäuse verklebt.

13. Verfahren zum Einstellen einer Orthese zum Unterstützen mindestens eines Körperteils einer Person, das die folgenden Schritte, zur Ausführung in geeigneter Reihenfolge, umfasst:
(a) Bereitstellen einer Orthese gemäß einem der Ansprüche 1 bis 12, wobei die Orthese umfasst:
- ein Gehäuse;
- mindestens ein verformbare Element, das in dem Gehäuse angeordnet ist, wobei das verformbare Element mit einem Medium gefüllt ist, das durch Aktivierung härtbar ist, oder mit einem härtbaren Medium befüllbar ist, zum unterstützen des Körperteils der Person nach dem Härten des Mediums,
wobei das Gehäuse eine Kontaktoberfläche zum Anliegen an dem Körperteil aufweist, wobei das verformbare Element nur über einem Teil der Kontaktoberfläche und nicht über einem anderen Teil davon angeordnet ist,
(b) Aktivieren des härtbaren Mediums oder Befüllen des verformbaren Elements mit dem härtbaren Medium und
(c) Härtenlassen des härtbaren Mediums.

14. Verfahren gemäß Anspruch 13, wobei Schritt (b) und/oder Schritt (c) ausgeführt wird, während der Körperteil der Person von der Orthese unterstützt wird.

## Revendications

1. Orthèse destinée à supporter une partie au moins du corps d'une personne, comprenant :
- une enveloppe (2) ;
- au moins un élément qui peut être déformé (6) disposé dans l'enveloppe, l'élément qui peut être déformé étant rempli avec un milieu qui peut durcir par activation, ou pouvant être rempli avec un milieu qui peut durcir, destiné à supporter la partie du corps de la personne après le durcissement du milieu ;
**caractérisée en ce que** :
l'enveloppe (2) présente une surface de contact destinée à se trouver contre la partie du corps, dans laquelle l'élément qui peut être déformé (6) est disposé sur une partie seulement de la surface de contact, et non pas au-dessus d'une autre partie de celle-ci.

2. Orthèse selon la revendication 1, dans laquelle l'enveloppe comprend un élément de répartition de la pression.

3. Orthèse selon la revendication 2, dans laquelle l'enveloppe comprend un élément raide qui est connecté ou qui peut être connecté à l'élément de répartition de la pression, et dans laquelle l'élément qui peut être déformé est disposé, ou peut être disposé entre l'élément de répartition de la pression et l'élément raide.

4. Orthèse selon l'une quelconque des revendications précédentes, dans laquelle l'élément qui peut être déformé présente une forme adaptée à la partie du corps de la personne.

5. Orthèse selon l'une quelconque des revendications précédentes, dans laquelle l'élément qui peut être déformé comprend deux côtés qui sont disposés à l'opposé l'un de l'autre, et qui sont connectés l'un à l'autre le long de leur périphérie.

6. Orthèse selon la revendication 5, dans laquelle les deux côtés sont connectés l'un à l'autre à des emplacements déterminés le long de la surface des deux côtés.

7. Orthèse selon l'une quelconque des revendications précédentes, dans laquelle l'élément présente au moins un pli périphérique.

8. Orthèse selon la revendication 7, dans laquelle une extrémité dirigée vers l'intérieur du pli, est connectée à une partie de l'élément qui peut être déformé.

9. Orthèse selon l'une quelconque des revendications précédentes, dans laquelle l'élément qui peut être déformé présente au moins une ouverture de sortie d'air.

10. Orthèse selon l'une quelconque des revendications précédentes, comprenant des moyens de fixation destinés à fixer l'élément qui peut être déformé sur l'enveloppe, de telle sorte que l'élément qui peut être déformé puisse être disposé de manière fixe à un emplacement déterminé dans l'enveloppe.

11. Orthèse selon la revendication 10, dans laquelle les moyens de fixation sont sélectionnés dans le groupe constitué par de la colle et un ruban auto-agrippant.

12. Orthèse selon la revendication 11, dans laquelle l'élément qui peut être déformé présente une ouverture d'évacuation destinée à évacuer une partie du milieu, de telle sorte que le milieu acheminé par l'intermédiaire de l'ouverture d'évacuation hors de l'élément qui peut être déformé, fasse adhérer l'élément qui peut être déformé à l'enveloppe.

13. Procédé destiné à régler une orthèse destinée à supporter une partie au moins du corps d'une personne, comprenant les étapes, à exécuter dans l'ordre approprié, consistant à :
(a) fournir une orthèse selon l'une quelconque des revendications 1 à 12, l'orthèse comprenant :
- une enveloppe ;
- au moins un élément qui peut être déformé disposé dans l'enveloppe, l'élément qui peut être déformé étant rempli avec un milieu qui peut durcir par activation, ou pouvant être rempli avec un milieu qui peut durcir, destiné à supporter la partie du corps de la personne après le durcissement du milieu ;
dans lequel l'enveloppe présente une surface de contact destinée à se trouver contre la partie du corps, dans lequel l'élément qui peut être déformé est disposé sur une partie seulement de la surface de contact, et non pas au-dessus d'une autre partie de celle-ci ;
(b) activer le milieu qui peut durcir, ou remplir l'élément qui peut être déformé avec un milieu qui peut durcir ; et
(c) permettre au milieu qui peut durcir de durcir.

14. Procédé selon la revendication 13, dans lequel l'étape (b) et / ou l'étape (c) sont exécutées tandis que la partie du corps de la personne porte contre l'orthèse.
